# FASCICULE DE BREVET EUROPEEN

(11) **EP 1 066 068 B1**
(45) Date de publication et mention de la délivrance du brevet: **12.07.2006**
(21) Numéro de dépôt: 99910578.6
(22) Date de dépôt: 01.04.1999
(51) Int. Cl.: A61M 1/28

(54) **DISPOSITIF DE DIALYSE PERITONEALE**
ANORDNUNG FÜR DIE PERITONEALDIALYSE
DEVICE FOR PERITONEAL DIALYSIS

(30) Priorité: 02.04.1998 CH 79198
(43) Date de publication de la demande: 10.01.2001
(73) Titulaire: DEBIOTECH S.A., 1004 Lausanne (CH)
(72) Inventeur: NEFTEL, Frédéric, CH-1000 Lausanne 9 (CH)
(74) Mandataire: Cardy, Sophie Marie
(86) Numéro de dépôt international: PCT/IB1999/000559
(87) Numéro de publication internationale: WO 1999/051287

(56) Documents cités:
- EP-A- 0 402 505
- EP-A- 0 498 382
- DE-A- 3 333 362

## Description

La présente invention se rapporte au traitement de l'insuffisance rénale et plus précisément à un dispositif de dialyse péritonéale.

La dialyse péritonéale utilise le péritoine du patient à titre de membrane semi-perméable pour filtrer le sang.

Lors d'une dialyse péritonéale, une solution aqueuse stérile (le dialysat) est administrée dans la cavité péritonéale. Cette dernière est séparée du flux sanguin par notamment le péritoine, de sorte que des échanges diffusifs et osmotiques peuvent avoir lieu entre le dialysat et le sang. Ces échanges résultent en une élimination des produits de dégradation, tels que l'urée, le potassium où la créatinine, qui sont normalement excrétés par les reins. La diffusion d'eau à travers le péritoine pendant la dialyse est appelée ultrafiltration, le volume d'eau perdu par le patient est appelé ultrafiltrat.

A l'origine, la dialyse péritonéale était caractérisée par le fait que l'on introduisait initialement un volume donné de dialysat dans la cavité péritonéale, on laissait par la suite les échanges diffusifs et osmotiques se produire pendant une durée déterminée puis enfin, on retirait le volume de dialysat dans son intégralité pour le remplacer par un nouveau volume de dialysat.

Par la suite, la dialyse péritonéale s'est automatisée, ce qui a donné lieu à la méthode APD (Automated Péritonéal Dialysis) où une machine administre et retire le dialysat. Ce genre d'opération peut se produire plusieurs fois, ce que l'on nomme la méthode CCPD (Continuous Cycling Péritonéal Dialysis).

L'automatisation permet par exemple d'effectuer la dialyse durant la nuit, lorsque le patient dort.

La demande de brevet EP 402 505 A (A. Peabody) correspond à un dispositif de dialyse péritonéale à cycle continu. Le système est équipé de détecteurs de pression pour mesurer le volume d'ultrafiltrat. En injectant une solution de glucose, on fait varier directement l'osmolarité qui permettra d'effectuer des mouvements d'échanges et donc d'éliminer les produits de dégradation. Ce dispositif basé sur la variation du gradiant osmotique s'est avéré limité par rapport à la dialyse de type "Tidal".

De même, la demande de brevet DE 33 33 362 A (Fresenius A.G.) décrit également un dispositif basé sur la variation du gradiant osmotique. On assiste à un prélèvement de façon intermittente du liquide mais uniquement à titre de mesure de concentration osmotique de la substance active.

La dialyse péritonéale de type « Tidal » ou TPD (Tidal Peritoneal Dialysis) est caractérisée par une série de cycles administration-latence-retrait du dialysat, mais contrairement aux méthodes décrites précédemment, il n'y a pas renouvellement intégral du volume de dialysat à chaque cycle. De manière cyclique, seule une fraction du volume total est renouvelée, à l'exception toutefois de la dernière phase du cycle où le volume dans son intégralité est retiré.

La demande WO 95/27520 décrit notamment un dispositif pour dialyse péritonéale. Le volume changé reste constant au cours des échanges et peut être de l'ordre de 300 ml, soit moins de 15 % du volume initialement administré.

Avec de tels volumes, il est possible d'atteindre des cycles de haute fréquence.

Le fait d'atteindre une haute fréquence présente l'avantage de renouveler partiellement le dialyse fréquemment afin de maintenir une meilleure qualité du dialysat, d'où une purification améliorée du sang.

La demande de brevet EP 498 382 A (A. Peabody) décrit un dispositif utilisable pour la dialyse de type "Tidal". Les paramètres d'échange de dialysat ne varient pas. La fréquence et l'amplitude des volumes échangés sont constants, seul le volume résiduel dans la cavité augmente au cours du temps. L'augmentation de volume observée peut être qualifiée "d'accidentelle" car non programmée, elle résulte simplement de l'augmentation de l'ultrafiltrat. Ainsi, ce document n'envisage pas un système de variation du volume résiduel.

Bien que les dispositifs de dialyse péritonéale de l'état de la technique donnent une certaine satisfaction, il subsiste cependant un certain nombre d'inconvénients.

Le changement constant d'un même volume au cours du traitement entraîne une consommation importante de liquide de dialyse et une durée prolongée du traitement.

Par ailleurs, une fois le volume initial administré, bien que de petits volumes soient périodiquement changés, la qualité du dialysat dans la cavité péritonéale diminue au cours du temps, ce qui entraîne une baisse de la purification au cours du temps.

La présente invention vise à remédier notamment aux inconvénients mentionnés précédemment.

A cet effet, l'invention propose un dispositif conforme à la revendication 1 et les sous-revendications 2 à 21.

Elle est caractérisée par le fait qu'elle est essentiellement constituée d'un dispositif muni d'un système permettant de faire varier les paramètres d'échange du dialysat au cours du temps afin de maintenir une qualité optimale du dialysat tout en optimisant les volumes d'échange de façon à minimiser la consommation totale de liquide de dialyse.

Afin de réaliser ce but, la fréquence des cycles d'échange peut être variée. Elle peut être basse en début de traitement et augmenter au cours du traitement lorsque la qualité du dialysat dans la cavité péritonéale diminue.

De même, le volume changé peut varier au cours du traitement. E peut être relativement faible en début de traitement et augmenter au cours du traitement lorsque la qualité du dialysat dans la cavité péritonéale diminue.

La présente invention vise également à faire varier le volume total de dialysat dans la cavité péritonéale au cours du temps. Ce volume peut par exemple augmenter en cours de traitement. Ce cas de figure peut être réalisé en administrant des volumes d'échange supérieurs aux volumes d'échange retirés.

La période de latence, durée entre l'administration d'un volume d'échange et le retrait d'un volume d'échange, peut être variable. Elle peut être longue en début de traitement et diminuer au cours du traitement de façon à compenser au moins partiellement la baisse de purification du sang dû à la moindre qualité du dialysat.

Le débit lors d'un échange peut être variable. Il peut être faible en début de traitement et augmenter au cours du temps pour les mêmes motifs qu'évoqués précédemment.

L'optimisation des échanges vise à obtenir une meilleure filtration et un ultrafiltrat plus important tout en diminuant le volume total du dialysat nécessaire et la durée du traitement.

Le système de variation du dispositif de l'invention est programmé en utilisant des paramètres d'échange qui sont établit d'après une optimisation tenant compte des paramètres propres à chaque patient (courbes de filtration). A titre d'exemple, le volume de dialysat total utilisé ou la durée du traitement peut être considéré lors d'une telle optimisation.

Il est notamment possible de créer des modèles mathématiques permettant de réaliser une telle optimisation en tenant compte, entre autres, des paramètres de filtration des patients considérés.

Il est également possible de réchauffer le liquide destiné à être administré au patient au cours des échanges de dialysats par récupération de la chaleur provenant du liquide de dialysat retiré du patient à travers un système d'échange calorique (14). Pour se faire, on peut par exemple faire passer le liquide retiré dans un tube qui contient lui-même un autre tube d'amenée de liquide de dialysat neuf qui doit être réchauffé.

On peut également augmenter la surface d'échange entre les deux liquides en utilisant une tubulure en serpentin qui passe dans une poche contenant le liquide extrait du patient.

Un système par exemple de valves anti-retour permet la séparation des liquides en provenance du patient et destiné au patient au travers de ce système d'échange calorique.

La figure 1 illustre schématiquement la baisse de la purification au cours du temps lorsque l'on utilise les dispositifs de l'état de la technique pendant un cycle.

La figure 2 illustre schématiquement les échanges de volumes au cours du traitement conformément à la présente invention.

La figure 3 représente un mode de réalisation du dispositif selon l'invention.

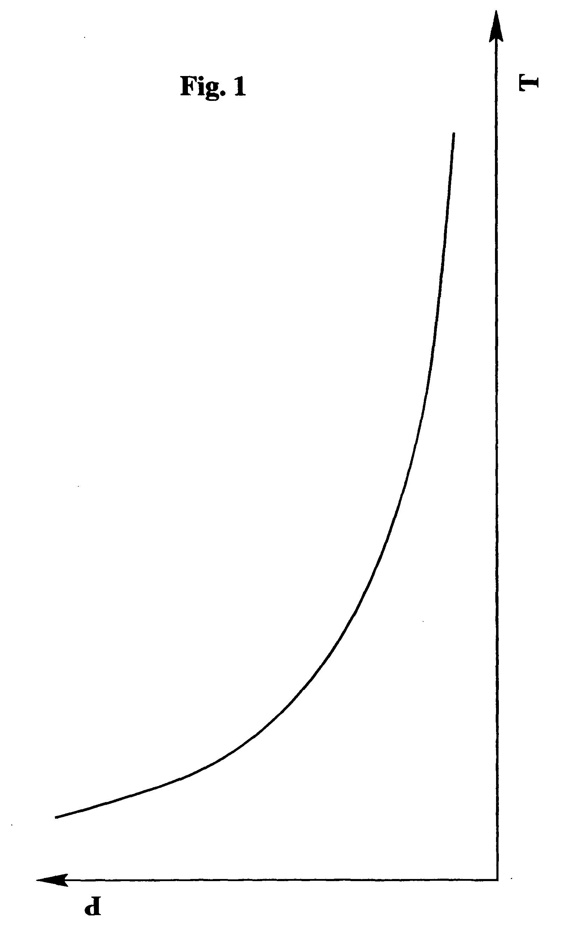

Le graphe de la figure 1 représente le niveau de purification (ordonnée) en fonction du temps (abscisse). Comme on peut le constater, pour un volume de base inchangé au cours du traitement, le niveau de purification baisse de manière exponentielle au cours du temps.

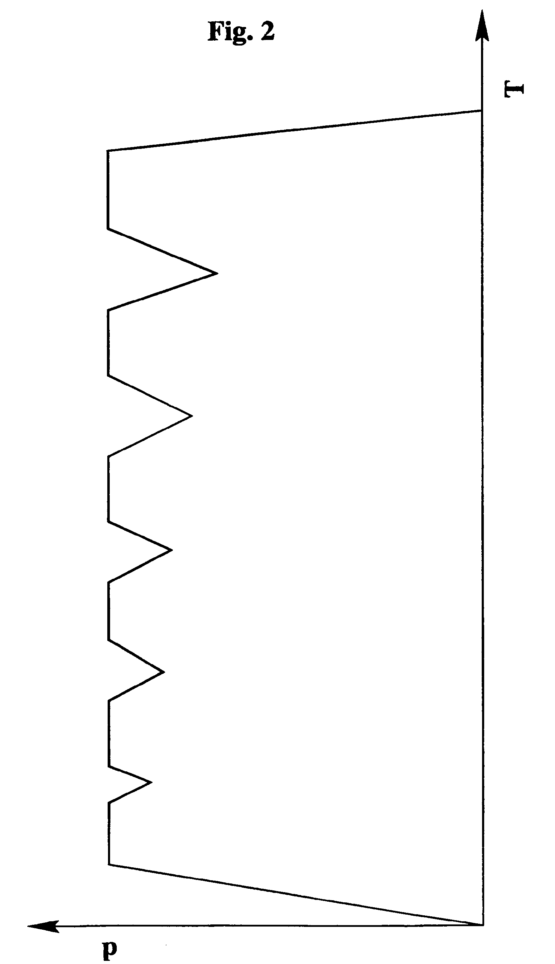

Le graphe de la figure 2 représente le volume de dialysat (ordonnée) dans la cavité péritonéale au cours du temps (abscisse). Dans ce mode de réalisation, on augmente la fréquence des échanges de fraction du dialyse ainsi que le volume de ces mêmes fractions au cours du temps, ce qui a pour effet de réduire notablement la baisse de purification décrite dans le graphe 1 tout en optimisant la quantité totale de dialysat nécessaire.

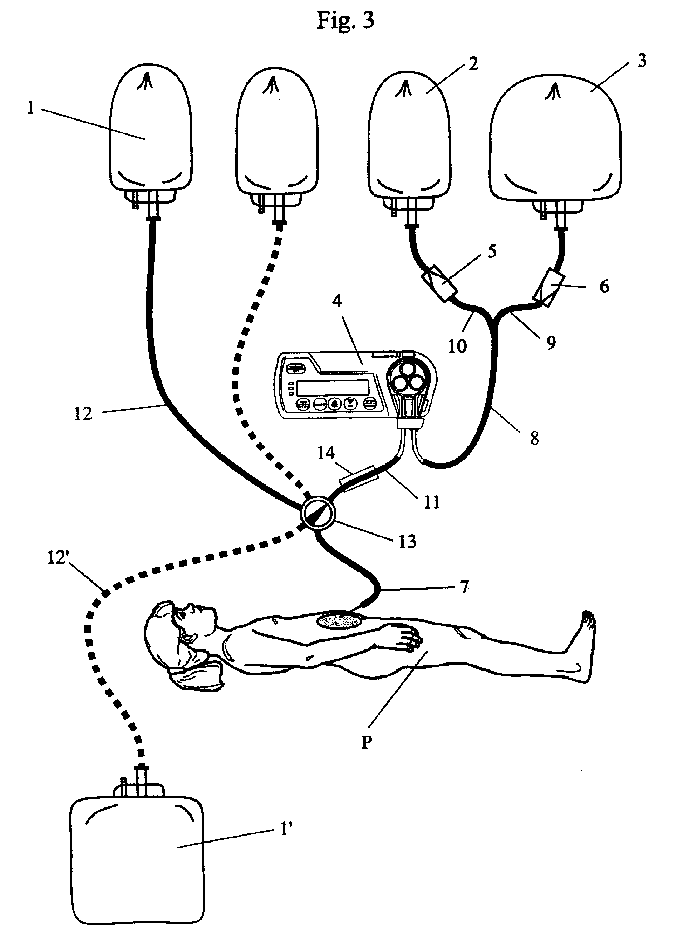

Le dispositif de la figure 3 est constitué d'un premier système de tubulures (7,12) permettant d'administrer un volume de base qui se situe dans un premier réservoir (1). Le même système de tubulure, mais positionné différemment (7,12') peut être utilisé pour récupérer le volume restant à la fin du traitement.

Un deuxième système de tubulures (7-11) est prévu pour administrer et retirer les volumes d'échange. Un réservoir d'administration (3) contient le dialysat à administrer et un autre réservoir de récupération (2) récupère le dialysat utilisé.

Le réservoir d'administration (3) est muni à sa sortie d'une valve anti-retour (6) qui l'empêche de recevoir le dialysat utilisé.

Le réservoir de récupération (2) est muni à son entrée d'une valve anti-retour (5) qui évite une sortie accidentelle du dialysat utilisé du réservoir de récupération.

Le liquide circulant dans le deuxième système de tubulures est acheminé par une pompe péristaltique (4).

Les deux systèmes de tubulures sont reliés entre eux par un robinet à plusieurs voies (13).

Le dispositif selon l'invention fonctionne de la manière suivante :

Le robinet à plusieurs voies (13) est disposé de façon à permettre l'administration du volume de base, qui se situe dans le premier réservoir (1), vers la cavité péritonéale du patient (P). Le premier réservoir (1) est disposé de telle manière que l'acheminement du liquide se produit par gravité.

Une fois la cavité péritonéale remplie, le robinet à plusieurs voies (13) est disposé de façon à mettre en relation le deuxième système de tubulures (7-11) avec la cavité péritonéale.

Lors du premier échange de volume, la pompe péristaltique (4) est actionnée de façon à retirer un volume d'échange. La disposition des valves anti-retour (5, 6) permet d'acheminer le dialysat utilisé dans le réservoir de récupération (2).

Une fois cette étape effectuée, la pompe péristaltique est actionnée de façon à prélever un volume d'échange dans le réservoir d'administration (3) pour l'acheminer vers la cavité péritonéale.

Un tel système peut notamment fonctionner avec une pompe péristaltique à faible débit (de l'ordre de 2 à 3 litres/heures) tel que décrite dans le brevet français FR 89 03 234.

Ce type d'opération s'effectue plusieurs fois au cours du traitement, en variant les paramètres conformément aux explications décrites précédemment.

En fin de traitement, le robinet à plusieurs voies (13) est disposé de façon à mettre en relation le premier système de tubulures (7,12) avec la cavité péritonéale. Le réservoir (1) est placé de façon à récupérer le volume restant par gravité.

Il faut relever que le mode de réalisation de l'invention décrit ci-dessus ne saurait limiter la portée de l'objet de cette dernière. Tout dispositif de dialyse péritonéale faisant varier les paramètres d'échange de fractions du dialysat au cours du temps doit être considéré comme faisant partie de la présente invention.

## Revendications

1. Dispositif de dialyse péritonéale adapté pour fonctionner en régime TPD (*Tidal Peritoneal Dialysis),* **caractérisé par le fait qu'**il comprend un système (4) de variation des paramètres d'échange du dialysat au cours du temps programmé et en utilisant lesdits paramètres d'échange qui sont établis en tenant compte de paramètres de filtration propres à chaque patient considéré, les paramètres d'échange du dialysat variant entre un cycle d'échange et le suivant et étant constitués par l'un ou plusieurs des paramètres suivants: fréquence des cycles d'échange, volume d'échange, volume total du dialysat, période de latence et débit.

2. Dispositif selon la revendication 1, **caractérisé par le fait que** le système (4) de variation est conçu de manière à faire varier la durée entre deux cycles d'échange de fractions du dialysat.

3. Dispositif selon la revendication 1 ou 2, **caractérisé par le fait que** le système (4) de variation est conçu de manière à faire varier le volume de la fraction du dialysat au cours des échanges.

4. Dispositif selon la revendication 3, **caractérisé par le fait que** le volume administré n'est pas identique au volume retiré.

5. Dispositif selon la revendication 4, **caractérisé par le fait que** le volume administré est inférieur au volume retiré.

6. Dispositif selon l'une quelconque des revendications précédentes, **caractérisé par le fait que** le système (4) de variation est conçu de manière à ce que le débit des échanges est variable d'un échange à l'autre.

7. Dispositif selon l'une quelconque des revendications précédentes, **caractérisé par le fait que** le système (4) de variation est programmé en se basant sur le volume de dialysat total disponible.

8. Dispositif selon l'une quelconque des revendications précédentes, **caractérisé par le fait que** le système (4) de variation est programmé en se basant sur la durée maximale totale de la dialyse.

9. Dispositif selon l'une quelconque des revendications précédentes, **caractérisé par le fait qu'**il se présente sous la forme de deux systèmes de tubulures, le premier (7,12) permettant d'introduire et de retirer le volume de base, le deuxième (7,11) permettant d'assurer les échanges de fraction de dialysat.

10. Dispositif selon la revendication 9, **caractérisé par le fait que** le liquide du premier système de tubulures (7,12) est acheminé par gravité alors que le liquide du deuxième système de tubulures (7,11) est acheminé par pompage.

11. Dispositif selon la revendication 9 ou 10, **caractérisé par le fait que** le deuxième système de tubulures est doté d'un réservoir (3) pour administrer des fractions de dialyse et d'un réservoir (2) pour récupérer des fractions de dialysat.

12. Dispositif selon la revendication 11, **caractérisé par le fait que** les deux réservoirs (3,2) sont munis de valves anti-retour (6,5).

13. Dispositif selon l'une quelconque des revendications 9 à 12, **caractérisé par le fait qu'**il est muni d'un échangeur de chaleur permettant de réchauffer le liquide administré par le liquide retiré.

14. Dispositif selon l'une quelconque des revendications 10 à 13, **caractérisé par le fait qu'**il est muni d'une micropompe péristaltique programmable (4).

15. Dispositif selon l'une quelconque des revendications précédentes, **caractérisé par le fait qu'**un robinet (13) à plusieurs voies est actionné automatiquement par la pompe (4) entre le premier système de tubulures (7,12) et le deuxième système de tubulures (7,11) en fonction des cycles d'échanges souhaités.

16. Dispositif selon l'une quelconque des revendications précédentes, **caractérisé en ce que** la fréquence des cycles d'échange augmente au cours du temps.

17. Dispositif selon l'une quelconque des revendications précédentes, **caractérisé en ce que** le volume d'échange (E) augmente au cours du temps.

18. Dispositif selon l'une quelconque des revendications précédentes, **caractérisé en ce que** le volume total de dialysat dans la cavité péritonéale augmente au cours du temps.

19. Dispositif selon la revendication 18, **caractérisé en ce que** le volume total de dialysat dans la cavité péritonéale augmente par le fait que l'on administre des volumes d'échange supérieurs aux volumes d'échange retirés.

20. Dispositif selon l'une quelconque des revendications précédentes, **caractérisé en ce que** la période de latence, entre l'administration d'un volume d'échange et le retrait d'un volume d'échange, diminue au cours du temps.

21. Dispositif selon l'une quelconque des revendications précédentes, **caractérisé en ce que** lors de l'administration d'un volume d'échange et/ou lors du retrait du volume d'échange de dialysat, le débit augmente au cours du temps.

## Claims

1. A peritoneal dialysis device adapted to function in tidal peritoneal dialysis (TPD) mode, the device being **characterised by** the fact that it is provided with a variation system (4) for varying the parameters of dialysate exchange over the programmed time by using said exchange parameters which are established by taking account of the filtration parameters specific to each patient under consideration, the exchange dialysate parameters varying between two successive exchange cycles and being constituted by one or more of the following parameters: frequency of the exchange cycles, exchange volume, total volume of the dialysate, pause period and flowrate.

2. A device according to claim 1, **characterised by** the fact that the variation system (4) is designed in such a manner as to vary the duration between two cycles for exchanging fractions of dialysate.

3. A device according to claim 1 or 2, **characterised by** the fact that the variation system (4) is designed in such a manner as to vary the volume of the dialysate fraction during exchanges.

4. A device according to claim 3, **characterised by** the fact that the volume administered is not identical to the volume removed.

5. A device according to claim 4, **characterised by** the fact that the volume administered is less than the volume removed.

6. A device according to any one of the preceding claims, **characterised by** the fact that the variation system (4) is designed in such a manner that the flowrate of the exchanges is variable from one exchange to another.

7. A device according to any one of the preceding claims, **characterised by** the fact that the variation system (4) is programmed on the basis of the total volume of dialysate available.

8. A device according to any one of the preceding claims, **characterised by** the fact that the variation system (4) is programmed on the basis of the maximum total duration of the dialysis.

9. A device according to any one of the preceding claims, **characterised by** the fact that it is presented in the form of two systems of tubes, the first (7, 12) enabling an initial volume to be introduced and removed, and the second (7, 11) enabling fractions of dialysate to be exchanged.

10. A device according to claim 9, **characterised by** the fact that the liquid in the first system of tubes (7, 12) is conveyed by gravity while the liquid in the second system of tubes (7, 11) is conveyed by pumping.

11. A device according to claim 9 or 10, **characterised by** the fact that the second system of tubes is provided with a reservoir (3) for administering dialysis fractions and with a reservoir (2) for recovering fractions of dialysate.

12. A device according to claim 11, **characterised by** the fact that both reservoirs (3, 2) are provided with non-return valves (6, 5).

13. A device according to any one of claims 9 to 12, **characterised by** the fact that it is provided with a heat exchanger enabling the liquid for administration to be warmed by the liquid that has been removed.

14. A device according to any one of claims 10 to 13, **characterised by** the fact that it is provided with a programmable peristaltic micropump (4).

15. A device according to any one of the preceding claims, **characterised by** the fact that a multi-port valve (13) is actuated automatically by the pump (4) between the first system of tubes (7, 12) and the second system of tubes (7, 11) as a function of the desired exchange cycles.

16. A device according to any one of the preceding claims, **characterised in that** the frequency of the exchange cycles increases over time.

17. A device according to any one of the preceding claims, **characterised in that** the exchange volume (E) increases over time.

18. A device according to any one of the preceding claims, **characterised in that** the total volume of dialysate in the peritoneal cavity increases over time.

19. A device according to claim 18, **characterised in that** the total volume of dialysate in the peritoneal cavity increases by the fact that the exchange volumes administered are greater than the exchange volumes removed.

20. A device according to any one of the preceding claims, **characterised in that** the pause period, i.e. the time between the administration of an exchange volume and the removal of an exchange volume, diminishes over time.

21. A device according to any one of the preceding claims, **characterised in that** during the administration of an exchange volume and/or the removal of the exchange volume of the dialysate, the flowrate increases over time.

## Patentansprüche

1. Peritoneale Dialysevorrichtung, ausgelegt um im TPD-Betrieb (Tidale Peritoneale Dialyse) zu arbeiten, **gekennzeichnet durch** die Tatsache, daß sie ein System (4) zur zeitlichen Variation der Dialysataustauschparameter umfaßt, die programmiert ist, unter Verwendung der Austauschparameter, die gebildet sind unter Berücksichtigung von eigenen Filtrationsparametern für jeden betreffenden Patienten, wobei die Austauschparameter des Dialysats von einem Austauschzyklus zum folgenden varüeren und **durch** einen oder mehrere der folgenden Parameter gebildet werden: Frequenz der Austauschzyklen, Austauschvolumen, Gesamtvolumen des Dialysats, Latenzzeit und Durchsatz.

2. Vorrichtung nach Anspruch 1, **gekennzeichnet durch** die Tatsache, daß das System (4) zur Variation vorgesehen ist, um die Dauer zwischen zwei Austauschzyklen von Dialysatfraktionen variieren zu lassen.

3. Vorrichtung nach Anspruch 1 oder 2, **gekennzeichnet durch** die Tatsache, daß das System (4) zur Variation in einer Weise vorgesehen ist, daß das Volumen der Fraktion des Dialysats im Laufe der Austausche varüert wird.

4. Vorrichtung nach Anspruch 3, **gekennzeichnet durch** die Tatsache, daß das verabreichte Volumen nicht mit dem abgezogenen Volumen identisch ist.

5. Vorrichtung nach Anspruch 4, **gekennzeichnet durch** die Tatsache, daß das verabreichte Volumen kleiner als das abgezogene Volumen ist.

6. Vorrichtung nach einem der vorhergehenden Ansprüche, **gekennzeichnet durch** die Tatsache, daß das System (4) zur Variation derart vorgesehen ist, daß der Durchsatz der Austausche variabel untereinander ist.

7. Vorrichtung nach einem der vorhergehenden Ansprüche, **gekennzeichnet durch** die Tatsache, daß das System (4) zur Variation basierend auf dem gesamten verfügbaren Dialysatvolumen programmiert ist.

8. Vorrichtung nach einem der vorhergehenden Ansprüche, **gekennzeichnet durch** die Tatsache, daß das System (4) zur Variation basierend auf der maximalen Gesamtdauer der Dialyse programmiert ist.

9. Vorrichtung nach einem der vorhergehenden Ansprüche, **gekennzeichnet durch** die Tatsache, daß sie in Form von zwei Röhrensystemen vorliegt, wobei das erste (7, 12) es ermöglicht, das Basisvolumen einzuführen und abzuziehen und das zweite (7, 11) es ermöglicht, die Dialysatfraktionsaustausche sicherzustellen.

10. Vorrichtung nach Anspruch 9, **gekennzeichnet durch** die Tatsache, daß die Flüssigkeit des ersten Röhrensystems (7, 12) **durch** Schwerkraft zugeleitet wird, während die Flüssigkeit des zweiten Röhrensystems (7, 11) **durch** Pumpen zugeleitet wird.

11. Vorrichtung nach Anspruch 9 oder 10, **gekennzeichnet durch** die Tatsache, daß das zweite Röhrensystem mit einem Tank (3), um Dialysefraktionen zu verabreichen und einem Tank (3), um Dialysatfraktionen zu gewinnen, versehen ist.

12. Vorrichtung nach Anspruch 11, **gekennzeichnet durch** die Tatsache, daß die beiden Tanks (3, 2) mit Antirücklaufventilen (6, 5) ausgerüstet sind.

13. Vorrichtung nach einem der Ansprüche 9 bis 12, **gekennzeichnet durch** die Tatsache, daß sie mit einem Wärmeaustauscher ausgerüstet ist, der es ermöglicht, die verabreichte Flüssigkeit **durch** die abgezogenen Flüssigkeit zu erwärmen.

14. Vorrichtung nach einem der Ansprüche 10 bis 13, **gekennzeichnet durch** die Tatsache, daß sie mit einer peristaltischen programmierbaren Mikropumpe (4) ausgerüstet ist.

15. Vorrichtung nach einem der vorhergehenden Ansprüche, **gekennzeichnet durch** die Tatsache, daß ein Mehrwegehahn (13) automatisch **durch** die Pumpe (4) zwischen dem ersten Röhrensystem (7, 12) und dem zweiten Röhrensystem (7, 11) in Abhängigkeit der gewünschten Austauschzyklen betätigt wird.

16. Vorrichtung nach einem der vorhergehenden Ansprüche, **dadurch gekennzeichnet, daß** die Frequenz der Austauschzyklen sich im Laufe der Zeit erhöht.

17. Vorrichtung nach einem der vorhergehenden Ansprüche, **dadurch gekennzeichnet, daß** das Austauschvolumen (E) sich im Laufe der Zeit erhöht.

18. Vorrichtung nach einem der vorhergehenden Ansprüche, **dadurch gekennzeichnet, daß** das Gesamtdialysatvolumen in der Peritonealhöhle sich im Laufe der Zeit erhöht.

19. Vorrichtung nach Anspruch 18, **dadurch gekennzeichnet, daß** das Gesamtdialysatvolumen in der Peritonealhöhle sich durch die Tatsache erhöht, daß man höhere Austauschvolumen verabreicht als die abgezogenen Austauschvoluminen.

20. Vorrichtung nach einem der vorhergehenden Ansprüche, **dadurch gekennzeichnet, daß** die Latenzzeit zwischen der Verabreichung eines Austauschvolumens und dem Abziehen eines Austauschvolumens sich im Laufe der Zeit vermindert.

21. Vorrichtung nach einem der vorhergehenden Ansprüche, **dadurch gekennzeichnet, daß** bei der Verabreichung ein Austauschvolumen und/oder beim Abziehen des Dialysataustauschvolumens sich der Durchsatz im Laufe der Zeit erhöht.
